# EUROPEAN PATENT SPECIFICATION

(11) **EP 3 405 472 B1**
(45) Date of publication and mention of the grant of the patent: **01.01.2020**
(21) Application number: 17704099.5
(22) Date of filing: 20.01.2017
(51) Int. Cl.: C07H 15/04, C08B 31/10, C08B 31/12

(54) **ETHERIFICATION OF CARBOHYDRATES USING SUPERHEATED STEAM**
VERETHERUNG VON KOHLENHYDRATEN MITTELS ÜBERHITZTEM DAMPF
ÉTHÉRIFICATION DE GLUCIDES À L'AIDE DE VAPEUR SURCHAUFFÉE

(30) Priority: 22.01.2016 EP 16152456
(43) Date of publication of application: 28.11.2018
(73) Proprietor: Stichting Wageningen Research, 6708 PB Wageningen (NL)
(72) Inventor: SLAGHEK, Theodoor Maximiliaan, 3042 HT Rotterdam (NL); TIMMERMANS, Johannes Wilhelmus, 6714 GG Ede (NL); HAAKSMAN, Ingrid Karin, 3823 KG Amersfoort (NL); HOPMAN, Johannes Cornelis Petrus, 9641 GK Veendam (NL)
(74) Representative: V.O.
(86) International application number: PCT/NL2017/050040
(87) International publication number: WO 2017/126969

(56) References cited:
- GB-A- 815 358
- GB-A- 2 094 323

## Description

The invention is in the field of carbohydrate ethers.

Carbohydrate ethers are known, and are generally understood to be carbohydrates, which are functionalized on a hydroxy group of the carbohydrate with an organic group by a C-O-C bond. Examples of carbohydrate ethers are carboxymethyl starch ether and certain cationic starches. Such carbohydrates are made conventionally by etherification of a starch in a polar solution or suspension, usually water, with a functionalizing molecule, e.g. chloroacetic acid or 3-chloro-2-hydroxypropyl trimethyl ammonium chloride.

It has been proposed in European patent application 0 333 292 to carry out an etherification reaction under conditions which use less water, in which the reaction mixture must contain 5-40 wt.% of water. An organic acid is required to make this work. Also in GB2094323 and GB815358, methods for the etherification of starch are disclosed.

A drawback of known reactions is that often a large volume of solvent is used. Also, etherification is conventionally achieved in a polar solvent environment, which limits the identity of the molecule used for etherification to compounds which can withstand such conditions, and are capable of reacting under these conditions.

A further drawback is that often, large quantities of etherification agent are necessary which is costly and leads to the production of large amounts of salt as byproduct, which results in a high environmental burden. Furthermore, the maximum degree of substitution (DS) which can be attained is usually low. Also, it is often required to add large quantities of salt during the reaction, such as up to 10 wt.% sodium chloride or sodium sulfate, in order to retain the starch granular structure.

There is a need for novel carbohydrate ethers, in particular starch ethers, which can be made economically with a decreased environmental load and a high DS. Also, there is a need for carbohydrate ethers having ether groups which could hitherto not be introduced into the carbohydrate molecule, in order to establish starch ethers with novel properties. The present invention provides a novel process for the preparation of carbohydrate ethers, which fulfills these needs.

The present invention pertains to a method for the etherification of a carbohydrate, comprising subjecting the carbohydrate to superheated steam under alkaline conditions in the presence of an etherification agent to obtain a carbohydrate ether, where the etherification is carried out in the absence of a solvent.

Etherification of carbohydrates is defined as forming a C-O-C bond between the hydroxyl group of a carbohydrate and a C-atom of an etherification agent. The C-atom of the etherification agent which is to be coupled to the hydroxyl group does not also comprise a carbonyl group (C=O), and preferably, the C-atom of the etherification agent which is coupled to the hydroxyl group further comprises only single bonds to C and H atoms.

Carbohydrates which may be etherified in the context of the present invention are not particularly limited, and include for example monosaccharides, disaccharides, oligosaccharides and polysaccharides. Also, common analogues of carbohydrates, such as amino or acylamino carbohydrates, acylated carbohydrates, uronic acids, etc., can be treated with the process of the invention. Dry, granular carbohydrates generally comprise some water, such as 0-25 wt.% water, usually 1 - 20 wt.%. The dry weight of a carbohydrate is the weight of the carbohydrate without any included water.

Examples of monosaccharides include glucose, xylose, galactose, fructose and the like. Disaccharides include e.g. sucrose, maltose, lactose, lactobionic acid etc. Oligosaccharides include galacto-oligosaccharides (a- or β-), fructo-oligosaccharides, malto-oligosaccharides, mixed oligosaccharides, and the like. Polysaccharides include starch from any source, such as wheat, maize, rice, potato, cassava, etc, including starch fractions or variants such as high-amylose starch or high-amylopectin starch, hydrolysates, etc., gums and other polysaccharides. Polysaccharides furthermore include other glucans (e.g. pullulan, dextran, alternan, microcrystalline cellulose), xyloglucans (e.g. tamarind), galactans, mannans, gluco-mannans, especially galactomannans (e.g. guar), fructans (e.g. inulin), arabans, xylans, arabinoxylans, arabinogalactans, galacturonans (including pectins), (hetero)-glucuronans (including gellan, xanthan, and the like) etc., as well as combinations thereof.

Preferably, the carbohydrate is a disaccharide, oligo- or polysaccharide. More preferably the carbohydrate is a polysaccharide having an average molecular weight of at least 1,500 Da (DP of at least 10), more preferably at least 10,000 (DP of at least 60), and may be as high as 10 MDa or even up to 100 MDa. The starch or other polysaccharide may be used in its native form, or it may be a polysaccharide derivative, such as a carboxymethylated, oxidised or hydroxyalkylated polysaccharides, as will be discussed further below.

Polysaccharides are preferred. Among polysaccharides, cellulose and starch are preferred, wherein starch is particularly preferred. Starch in this context may be modified or unmodified, and in case it is modified, it may have been degraded, such as by enzymatic or acid degradation, or by oxidation. Furthermore, the starch may have been crosslinked and/or modified by etherification, esterification or amidation. In case starch is used in an etherification according to the invention, it is further preferably in granular form when subjected to etherification.

Further preferably, the starch is a plant starch, such as a potato, pea, wheat, maize, rice or cassava (tapioca) starch. Further preferably, the starch is a root- or tuber starch, preferably a tapioca or potato starch, most preferably a potato starch.

Starch naturally comprises amylose and amylopectin, and there are no restrictions to the relative quantities of either compound in the starch for use in the present method. The relative amounts of amylose and amylopectin vary depending inter alia on the botanical source of the starch. As such, amylose-rich starch (also referred to as high amylose starch), amylopectin-rich starch (also referred to as waxy starch), as well as starch with any other ratio of amylose to amylopectin can be used in the present invention. In a particularly preferred embodiment, amylopectin-rich starch is used, such as for example an amylopectin-rich potato starch. For some ratios between amylose and amylopectin, it can be necessary to use starch from a genetically modified plant or a mutant plant.

The etherification agent is a compound which is capable of ether formation upon reaction with a hydroxyl group. This capability preferably stems from the presence in the etherification agent of an epoxide functional group, or from the presence of a carbon substituted with a leaving group such as iodide, bromide, chloride, triflate, tosylate, mesylate or carbonate, preferably iodide, chloride or bromide, most preferably chloride. The size of the etherification agent is not particularly limited. If the etherification agent is an epoxide, the etherification agent is preferably an agent comprising 2-32 carbon atoms. If the etherification agent is an agent which is substituted with a leaving group, it comprises preferably 1-32 carbon atoms, excluding carbon atoms in the leaving group(s), if any. Preferably, the etherification agent comprises an epoxide functional group.

The carbon comprising the leaving group does not further comprise groups which render it impossible in the present method for the leaving group to actually act as a leaving group. Thus, preferably, the carbon comprising the leaving group is a primary or secondary carbon atom, more preferably a primary carbon atom. Further preferably, the carbon comprising the leaving group does not comprise double or triple bonds.

The carbon substituted with a leaving group of the etherification agent may further comprise three single bonded atoms or groups of atoms, such as hydrogen atoms. Preferably, the carbon comprising the leaving group comprises, as single bonded group of atoms, a C₁ - C₃₁ linear, cyclic or branched, saturated or unsaturated alkyl group. Also, the linear, cyclic or branched, saturated or unsaturated alkyl group of the etherification agent may comprise aromatic rings and/or fused ring systems as well as ether, ester and/or amide bonds in or on a chain portion. Also, the linear, cyclic or branched, saturated or unsaturated alkyl group in the etherification agent may carry further functional groups such as acid, ester, amine, amide, thiol, and hydroxyl groups. Also, in case the carbon atom bearing the leaving group is a secondary carbon atom, the C₁ - C₃₁ linear, cyclic or branched, saturated or unsaturated alkyl group as defined above may be present as two separate single-bonded groups of atoms.

In other preferred embodiments of the present invention, the etherification agent comprises an epoxide functional group. In this case, the etherification agent can be an alkyl oxide, such as a C₂ - C₃₂ alkyl oxide, preferably a C₂ - C₂₀ alkyl oxide. Alternatively, compounds which form an epoxide in situ under the reaction conditions, such as halohydrins, among which epichlorohydrin, can be used as an etherification agent in the present method.

The epoxide group may be located in a central portion of the etherification agent, such as in a cyclic portion or inside a linear chain portion. Preferably however, the epoxide group is an epoxide group located on an end portion of the etherification agent, such as for example a 1,2-epoxide. The remainder of the etherification agent is a linear, cyclic or branched, saturated or unsaturated alkyl group. Also, the linear, cyclic or branched, saturated or unsaturated alkyl group of the etherification agent may comprise aromatic rings and/or fused ring systems as well as ether, ester and/or amide bonds in or on a chain portion. Also, the linear, cyclic or branched, saturated or unsaturated alkyl group in the etherification agent may carry further functional groups such as acid, ether, ester, amine, amide, thiol, and hydroxyl groups, preferably ether, ester or amide groups.

In a much preferred embodiment, the etherification agent comprises an epoxide functional group. Much preferred etherification agents are for example 2-ethylhexyl glycidyl ether (EHGE) or glycidyl 4-nonylphenyl ether (GNE).

The carbohydrate as defined above, is subjected to superheated steam in the presence of the etherification agent, also as defined above, in order to react the two compounds and form a carbohydrate ether. The carbohydrate ether comprises the carbohydrate portion, which is coupled through an ether bond to the substituent. The substituent is the portion of the etherification agent which is coupled to the carbohydrate after the reaction.

The substituent which results from reaction with an etherification agent comprising a leaving group as defined above is the full etherification agent minus the leaving group, which full etherification agent is coupled by the carbon atom formerly bearing the leaving group through an ether bond to the hydroxyl group of the carbohydrate.

The substituent which results from reaction with an etherification agent comprising an epoxide functional group is the etherification agent wherein the epoxide ring has been opened to form an ether bond to the carbohydrate by one of the carbon atoms of the former epoxide group, and which carries a hydroxyl group on the other carbon atom of the former epoxide group. Preferably, the carbon atom of the epoxide functional group which is least sterically hindered, such as for example the carbon atom which carries the most H-atoms, is the carbon atom onto which the ether bond to the carbohydrate is formed.

Subjecting the carbohydrate to the superheated steam in the presence of the etherification agent may be achieved by introducing the carbohydrate into a pressurized reactor which is capable of holding superheated steam. The carbohydrate and the etherification agent may be introduced into the reactor sequentially or simultaneously, and superheated steam may be introduced into the reactor prior to, simultaneous with or after the introduction of the carbohydrate and the etherification agent. Preferably, the superheated steam is introduced into the reactor after the introduction of the carbohydrate and the etherification agent. Further preferably, the mixture of carbohydrate and etherification agent is homogenized prior to the introduction of superheated steam.

The reaction between the carbohydrate and the etherification agent under the influence of superheated steam is preferably executed in a reactor. The skilled person is aware of many reactor types which may be used to effect reactions under superheated steam. Such reactors are preferably reactors which are capable of mixing powdered reactants under superheated steam conditions. Further preferably, reactors are equipped with both an entry and an exit point for superheated steam. Examples of suitable reactors are a batch fixed bed SHS reactor, a fluidized bed reactor, or an SHS spray dryer.

In the present method, the carbohydrate is subjected to superheated steam in the reactor under alkaline conditions. Alkaline conditions in this context are preferably achieved by subjecting the carbohydrate to a pretreatment with an alkaline agent prior to the etherification. The pretreatment preferably comprises subjecting the carbohydrate in powder form to the alkaline agent, preferably under continuous mixing.

The alkaline agent preferably comprises a hydroxide, such as an ammonium hydroxide or a metal hydroxide, such as calcium hydroxide or an alkali metal hydroxide. In a much preferred embodiment, the hydroxide is sodium hydroxide, potassium hydroxide, calcium hydroxide or lithium hydroxide, most preferably sodium or potassium hydroxide, most preferably sodium hydroxide. The pretreatment results in an alkali carbohydrate, which when subjected to superheated steam in the presence of an etherification agent effects alkaline conditions during the etherification.

Alternatively, the alkaline agent comprises a carbonate, such as sodium carbonate, or an organic base such as cyclic nitrogen heterocycles, particularly pyridine, or alkoxide salts, such as a sodium or potassium alkoxide, for example sodium ethoxide. Also, the alkaline agent may be a phosphate, such as a hydrogen phosphate.

The molar ratio between the alkaline agent and the carbohydrate to effect alkaline conditions is preferably 0.01 - 3 mol alkaline agent/mol carbohydrate monomer, preferably 0.02 - 1 mol alkaline agent/mol carbohydrate monomer, more preferably between 0.05 - 0.5 mol alkaline agent/mol carbohydrate monomer. The molar quantity of carbohydrate is determined by determining the dry weight of the carbohydrate, and dividing this dry weight by the molecular weight of the carbohydrate's monomeric unit.

Superheated steam, in the present context, is water vapor which is non-saturated. Non-saturated in this context means that the actual quantity of water in the steam at a certain temperature and pressure is lower than the maximum quantity of water that could be contained at that temperature, before water starts to condense. In other words, superheated steam of a certain temperature and pressure is steam which contains less water than it could contain at that temperature. This parameter is known as the relative humidity, which is the ratio between the actual quantity of water at a certain temperature and pressure and the maximum quantity of water in the steam at that temperature. The relative humidity in the present method should be less than 100 wt.%, in order to perform the present method for etherification of a carbohydrate using superheated steam. Described differently, superheated steam is steam heated to a temperature above the saturation temperature at a given pressure.

In a much preferred embodiment, the superheated steam effects a relative humidity ("RH") in the reactor of between 10 and 95 wt.%, preferably between 30 and 90 wt.%, more preferably between 50 and 80 wt.%. The relative humidity can be calculated from temperature and pressure, as is known by the skilled person. Tables with exemplary values of pressure and temperature from which the relative humidity can be derived, are widely available, for instance at the National Institute of Standards and Technology (NIST) website, or in the Heat Exchanger Design Handbook 5: Physical properties, edited by B. A. Bodling and M. Prescott and printed by Hemisphere Publishing Corporation. The reaction under superheated steam effects a lower water content during reaction than in known processes, such as slurry or oven processes, for etherification of starch.

In preferred embodiments, the superheated steam has a temperature of 50 - 300 °C, preferably from 75 to 250 °C, more preferably from 100 - 200 °C, even more preferably of 120 - 180 °C, even more preferably of 135 - 162 °C, even more preferably 140 - 158 °C.

The pressure in the present context is expressed as atm., which is equal to 1.01325×10⁵ N/m² (Pa). In preferred embodiments, the superheated steam has a pressure of 0.1 - 10 atm, preferably 0.5 - 8 atm., more preferably from 1 - 6 atm., even more preferably from 2 - 5 atm., most preferably 2.5 - 4 atm. The skilled persons knows how to combine a specific temperature with a specific pressure in order to obtain superheated steam having a relative humidity of below 100 wt.%.

In the present invention, the carbohydrate is subjected to superheated steam in the presence of an etherification agent but in the absence of a solvent. Any water included in the starch or in the steam is not considered a solvent. This effects a "dry" reaction, where the only compounds in the reactor are the carbohydrate, the etherification agent, and the superheated steam, and potentially an alkaline agent. This results in less energy expenditure which would otherwise be required for the removal of solvent, and also avoids the need for (sometimes environmentally unfriendly) solvents itself. Furthermore these conditions ensure the integrity of the starch granules is preserved, where otherwise granules would be damaged or disrupted at the elevated temperatures used. It is a distinct advantage of the present invention that the granular structure of the starch is retained without addition of large quantities of salt, such as sodium chloride or sodium sulfate, as is required using conventional slurry etherification.

In addition, dry conditions allow for easy obtaining of a pure starch ether, because the carbohydrate and the etherification agent react without formation of large quantities of by-products. This is in particular true for the case when the etherification agent comprises an epoxide functional group, where no by-products such as salts at all are formed, and the yield of starch ether is considerably increased. The product obtained may be already an substantially pure starch ether in the case of reaction with an epoxide containing etherification agent. In a preferred embodiment however, the carbohydrate ether obtained from the etherification method according to the present invention is washed after the reaction.

It is a further advantage of the present invention that using superheated steam as presently described, a higher degree of substitution (DS) can be attained than when using other methods of etherification using the same ratios of reactants, most notably the ratio between the carbohydrate and the etherification agent. This results in less etherification agent being required to attain a certain degree of substitution, with the concomitant cost- and environmental benefits. For example, using a 2:1 molar ratio between carbohydrate and etherification agent, conventional reaction conditions such as slurry reactions do not or barely result in measurable substitution, whereas the superheated steam treatment results in a DS which is considerably higher. Also, using oven conditions, a similar DS may be reached, but the time required to achieve that DS is much longer using traditional oven conditions than when using superheated steam. This may be due to the higher water content under oven conditions.

It is a further advantage that using superheated steam, carbohydrate ethers can be made which could not formerly be prepared. Thus, the scope of the superheated steam etherification is broader than the scope of for instance slurry etherifications.

It is preferred that the carbohydrate ether has a DS of from 0.005 to 2, more preferably from 0.01 to 1.

For the purpose of clarity and a concise description features are described herein as part of the same or separate embodiments, however, it will be appreciated that the scope of the invention may include embodiments having combinations of all or some of the features described.

The invention will now be illustrated by the following non-restricting examples.

### Examples

As an exemplary carbohydrate, an amylopectin-rich potato starch was used (Eliane™ 100, Avebe).

The carbohydrate was pretreated to result in an either acidic or alkaline carbohydrate, by stirring 1 kg carbohydrate (dry weight) for 15 hours in a Lödige powder mixer at 950 rpm after addition of 24.7 g of NaOH, or 85.2 g NaH₂PO₄ or 90.2 g adipic acid

The alkaline and acidic carbohydrates were used in the processes according to the invention using superheated steam, and in comparative processes using a slurry reaction or an oven reaction. It was found that the obtained DS under the same conditions was considerably higher, and/or the reaction time to attain the same DS was considerably shorter, when using superheated steam, than when using slurry- or oven conditions.

In the below, the DS has been determined by HPLC/ELSD on the basis of the assumption that the response factor for all components is more or less equal. This is applicable for measurements with an evaporating light scattering detector (ELSD). There are no reference standards for the exact components to be measured, so that the below reference standards were used. These reference standards were used in a conventional fashion:
Solutions of the used reference standards were prepared in a series of known concentrations, and the detector response of the reference standard was evaluated at each concentration. The detector response was thus usable as a measure for concentration, so that the detector response of the investigated samples allowed for knowing their concentration. The DS of the product was calculated after hydrolysis with 2M trifluoroacetic acid, for 1 hour at 100°C while stirring. After the pH of the hydrolysate was set to 6, the reference standard was added. The solution was diluted with methanol to obtain a concentration of 50% methanol. The amount of etherified glucose was measured with HPLC-ELSD, in relation to the reference standard.

The used reference standards were n-dodecyl β-D-maltoside for the calculation of the DS for glucose-EHGE, and octyl β-D-glucopyranoside for the DS of glucose-GNE.

### Example 1: Superheated steam reactions

The procedures are done in duplicate.

### Alkaline Eliane™ 100 and 2-ethylhexyl glycidyl ether

To 13 g (as is, moisture content 13.7 wt.%) alkaline amylopectin-rich potato starch (0.1 mol NaOH/mol anhydro-glucose), EHGE (0.2 mol/mol anhydro-glucose) is introduced and then homogenized. The mixture is transferred into the superheated steam reactor (SHS) for 2 hours. The SHS conditions are 150 °C and 3.3 atm. (RH 70 %).

### Acidic Eliane™ 100 and 2-ethylhexyl glycidyl ether

To 13 g (as is, moisture content 13.7 wt.%) phosphate loaded amylopectin-rich potato starch (0.1 mol NaH₂PO₄/mol anhydro-glucose), EHGE (0.2 mol/mol anhydro-glucose) is introduced and then homogenized. The mixture is transferred into the SHS for 2 hours. The SHS conditions are 150 °C and 3.3 atm. (RH 70 %).

### Acidic Eliane™ 100 and 2-ethylhexyl glycidyl ether

To 13 g (as is, moisture content 13.7 wt.%) adipic acid loaded amylopectin-rich potato starch (0.1 mol adipic acid/mol anhydro-glucose), EHGE (0.2 mol/mol anhydro-glucose) is introduced and then homogenized. The mixture is left overnight at room temperature. The mixture is transferred into the SHS for 2 hours. The SHS conditions are 150 °C and 3.3 atm. (RH 70 %).

The three above described procedures are repeated with glycidyl 4-nonyl-phenylether.

**Table 1 Overview products SHS reaction, 2 hours reaction time.**

| Starch | Epoxide | Average DS (2 hrs) |
|---|---|---|
| Alkaline starch | ethylhexyl glycidyl ether | 0.05 |
| | glycidyl 4-nonyl-phenylether | 0.007 |
| Phosphate starch | ethylhexyl glycidyl ether | ND |
| | glycidyl 4-nonyl-phenylether | ND |
| Adipic acid starch | ethylhexyl glycidyl ether | ND |
| | glycidyl 4-nonyl-phenylether | ND |

| | | |
|---|---|---|
| ND = not detected | | |

### Example 2: slurry reactions (comparative)

### Alkaline Eliane™ 100 and 2-ethylhexyl glycidyl ether

To 431 g water, 160 g sodium sulfate and 400 g (as is, moisture content 14.6 wt.%) amylopectin-rich potato starch was added. The suspension is stirred and sodium hydroxide (7.5g/kg starch) as 4 wt.% solution was added. EHGE (0.2 mol/mol anhydro-glucose) was introduced and the temperature was raised to 50 °C. The reaction was allowed to proceed 24 hours. The slurry was neutralized with 3 M sulfuric acid to pH 5.5. The sample was filtered, washed with water and dried.

The yield was 500g product, moisture content 31.2 wt.%, DS 0.001.

### Acidic Eliane™ 100 and glycidyl 4-nonyl-phenylether

To 431 g water, 160 g sodium sulfate and 400 g (as is, moisture content 14.6 wt.%) amylopectin-rich potato starch was added. The suspension is stirred and sodium hydroxide (7.5 g/kg starch) as 4.4 wt.% solution were added. GNE (0.2 mol/mol anhydro-glucose) is introduced and the temperature was raised to 50 °C. The reaction was allowed to proceed for 24 hours. The slurry was neutralized with 3 M sulfuric acid to pH 5.5. The sample was filtered, washed with water and dried.

The yield was 550 g product, moisture content 37.1 wt.%, DS 0 (not detected).

### Example 3: oven reactions (comparative)

### Alkaline Eliane™ 100 and 2-ethylhexyl glycidyl ether

To 175 g (as is, moisture content 13.7 wt.%) alkaline amylopectin-rich potato starch (0.1 mol NaOH/mol anhydo-glucose), 30 mL water was added and the resulting mixture was homogenized. Subsequently, EHGE (0.2 mol/mol anhydro-glucose) was introduced and the resulting mixture was homogenized. The mixture was transferred to a covered pot and left in an oven at 85 °C for 2, 4 or 24 hours, after which the DS was determined. The relative humidity in the oven is 100 wt.%.

### Acidic Eliane™ 100 and 2-ethylhexyl glycidyl ether

To 175 g (as is, moisture content 14.0 wt.%) phosphate loaded amylopectin (0.1 mol NaH₂PO₄/mol anhydro-glucose), 30 mL water was added and the resulting mixture was homogenized. Subsequently, EHGE (0.2 mol/mol anhydro-glucose) was introduced and the resulting mixture was homogenized. The mixture was transferred to a covered pot and left in an oven at 85 °C for 2, 4 or 24 hours, after which the DS was determined. The relative humidity in the oven is 100 wt.%.

### Acidic Eliane™ 100 and 2-ethylhexyl glycidyl ether

To 175 g (as is, moisture content 14.2 wt.%) adipic acid loaded amylopectin (0.1 mol adipic acid/mol anhydro-glucose), 30 mL water was added and the resulting mixture was homogenized. Subsequently, EHGE (0.2 mol/mol anhydro-glucose) was introduced and the resulting mixture was homogenized. The mixture was transferred to a covered pot and left in an oven at 85 °C for 2, 4 or 24 hours, after which the DS was determined. The relative humidity in the oven is 100 wt.%.

The three above described procedures were repeated with glycidyl 4-nonyl-phenylether.

**Table 2 Overview products oven reaction.**

| Starch | Epoxide | DS (2 hrs) | DS (4 hrs) | DS (24 hrs) |
|---|---|---|---|---|
| Alkaline starch | ethylhexyl glycidyl ether | 0.002 | 0.005 | 0.05 |
| | glycidyl 4-nonyl-phenylether | 0.001 | 0.001 | 0.004 |
| Phosphate starch | ethylhexyl glycidyl ether | ND | ND | ND |
| | glycidyl 4-nonyl-phenylether | ND | ND | ND |
| Adipic acid starch | ethylhexyl glycidyl ether | ND | ND | ND |
| | glycidyl 4-nonyl-phenylether | ND | ND | ND |

| | | | | |
|---|---|---|---|---|
| ND = not detected | | | | |

## Claims

1. A method for the etherification of a carbohydrate, comprising subjecting the carbohydrate to superheated steam under alkaline conditions in the presence of an etherification agent to obtain a carbohydrate ether, wherein the etherification is carried out in the absence of a solvent.

2. A method according to claim 1, wherein alkaline conditions are achieved by subjecting the carbohydrate to a pretreatment with an alkaline agent prior to the etherification.

3. A method according to claim 2, wherein the alkaline agent is a hydroxide, a carbonate or an organic base.

4. A method according to claim 2 or 3, wherein the ratio between the carbohydrate and the alkaline agent is 0.01 - 3 mol alkaline agent/mol carbohydrate monomer.

5. A method according to any of claims 1 - 4, wherein the superheated steam has relative humidity of between 10 and 95 wt.%.

6. A method according to any of claims 1 - 5, wherein the superheated steam has a temperature of 50 - 300 °C.

7. A method according to any of claims 1 - 6, wherein the etherification agent comprises an epoxide functional group or a carbon substituted with a leaving group such as iodide, bromide, chloride, triflate, tosylate, mesylate or carbonate.

8. A method according to any of claims 1 - 7, wherein the carbohydrate is starch.

9. A method according to claim 8, wherein the starch is granular.

## Patentansprüche

1. Verfahren zur Veretherung eines Kohlenhydrats, umfassend das Unterziehen des Kohlenhydrats einem überhitzten Dampf unter alkalischen Bedingungen in Gegenwart eines Veretherungsmittels, um einen Kohlenhydratether zu erhalten, wobei die Veretherung in Abwesenheit eines Lösungsmittels durchgeführt wird.

2. Verfahren nach Anspruch 1, wobei alkalische Bedingungen durch Unterziehen des Kohlenhydrats einer Vorbehandlung mit einem alkalischen Mittel vor der Veretherung erreicht werden.

3. Verfahren nach Anspruch 2, wobei das alkalische Mittel ein Hydroxid, ein Carbonat, oder eine organische Base ist.

4. Verfahren nach Anspruch 2 oder 3, wobei das Verhältnis zwischen dem Kohlenhydrat und dem alkalischen Mittel 0,01 - 3 Mol alkalisches Mittel/Mol Kohlenhydratmonomer ist.

5. Verfahren nach einem der Ansprüche 1 - 4, wobei der überhitzte Dampf eine relative Feuchtigkeit von zwischen 10 und 95 Gew.-% hat.

6. Verfahren nach einem der Ansprüche 1 - 5, wobei der überhitzte Dampf eine Temperatur von 50 - 300 °C hat.

7. Verfahren nach einem der Ansprüche 1 - 6, wobei das Veretherungsmittel eine epoxidfunktionelle Gruppe oder einen Kohlenstoff" substituiert mit einer Abgangsgruppe wie Iodid, Bromid, Chlorid, Triflat, Tosylat, Mesylat oder Carbonat, umfasst.

8. Verfahren nach einem der Ansprüche 1 - 7, wobei das Kohlenhydrat Stärke ist.

9. Verfahren nach Anspruch 8, wobei die Stärke körnig ist.

## Revendications

1. Procédé pour l'éthérification d'un hydrate de carbone, consistant à soumettre l'hydrate de carbone à de la vapeur surchauffée dans des conditions alcalines en présence d'un agent d'éthérification pour obtenir un éther d'hydrate de carbone, dans lequel l'éthérification est réalisée en l'absence d'un solvant.

2. Procédé selon la revendication 1, dans lequel les conditions alcalines sont réalisées en soumettant l'hydrate de carbone à un prétraitement avec un agent alcalin avant l'éthérification.

3. Procédé selon la revendication 2, dans lequel l'agent alcalin est un hydroxyde, un carbonate ou une base organique.

4. Procédé selon la revendication 2 ou 3, dans lequel le rapport entre l'hydrate de carbone et l'agent alcalin est de 0,01 - 3 moles d'agent alcalin/mole de monomère d'hydrate de carbone.

5. Procédé selon l'une quelconque des revendications 1-4, dans lequel la vapeur surchauffée présente une humidité relative de 10 à 95 % en masse.

6. Procédé selon l'une quelconque des revendications 1-5, dans lequel la vapeur surchauffée présente une température de 50 - 300°C.

7. Procédé selon l'une quelconque des revendications 1-6, dans lequel l'agent d'éthérification comprend un groupe fonctionnel époxyde ou un carbone substitué avec un groupe qui s'éloigne, tel que iodure, bromure, chlorure, triflate, tosylate, mésylate ou carbonate.

8. Procédé selon l'une quelconque des revendications 1-7, dans lequel l'hydrate de carbone est de l'amidon.

9. Procédé selon la revendication 8, dans lequel l'amidon est granulaire.
